# EUROPEAN PATENT SPECIFICATION

(11) **EP 3 314 912 B1**
(45) Date of publication and mention of the grant of the patent: **22.11.2023**
(21) Application number: 16813830.3
(22) Date of filing: 24.06.2016
(51) Int. Cl.: H04R 25/00, H04R 9/02, A61F 2/18, H04S 7/00, A61N 1/36

(54) **MAGNETIC RETENTION DEVICE**
MAGNETISCHE HALTEVORRICHTUNG
DISPOSITIF DE RETENUE MAGNÉTIQUE

(30) Priority: 26.06.2015 US 201562184993 P
(43) Date of publication of application: 02.05.2018
(73) Proprietor: Cochlear Limited, Macquarie University, NSW 2109 (AU)
(72) Inventor: GUSTAFSSON, Johan, New South Wales 2109 (AU); ANDERSSON, Marcus, New South Wales 2109 (AU); LEIGH, Charles Roger Aaron, New South Wales 2109 (AU)
(74) Representative: Grünecker Patent- und Rechtsanwälte PartG mbB
(86) International application number: PCT/IB2016/053787
(87) International publication number: WO 2016/207856

(56) References cited:
- EP-A2- 2 720 480
- WO-A1-99/39769
- JP-A- 2010 075 394
- US-A1- 2007 053 536
- US-A1- 2011 264 172
- US-A1- 2013 018 218
- US-A1- 2013 195 304
- US-A1- 2014 012 069

## Description

### Technical Field

The present invention relates to medical devices and particularly to bone conduction devices having an external component including two magnets for generating a magnetic retention force between the external component and an implanted magnet of a recipient.

### BACKGROUND

Hearing loss, which may be due to many different causes, is generally of two types: conductive and sensorineural. Sensorineural hearing loss is due to the absence or destruction of the hair cells in the cochlea that transduce sound signals into nerve impulses. Various hearing prostheses are commercially available to provide individuals suffering from sensorineural hearing loss with the ability to perceive sound. For example, cochlear implants use an electrode array implanted in the cochlea of a recipient to bypass the mechanisms of the ear. More specifically, an electrical stimulus is provided via the electrode array to the auditory nerve, thereby causing a hearing percept.

Conductive hearing loss occurs when the normal mechanical pathways that provide sound to hair cells in the cochlea are impeded, for example, by damage to the ossicular chain or the ear canal. Individuals suffering from conductive hearing loss may retain some form of residual hearing because the hair cells in the cochlea may remain undamaged.

Individuals suffering from conductive hearing loss typically receive an acoustic hearing aid. Hearing aids rely on principles of air conduction to transmit acoustic signals to the cochlea. In particular, a hearing aid typically uses an arrangement positioned in the recipient's ear canal or on the outer ear to amplify a sound received by the outer ear of the recipient. This amplified sound reaches the cochlea causing motion of the perilymph and stimulation of the auditory nerve.

In contrast to hearing aids, which rely primarily on the principles of air conduction, certain types of hearing prostheses commonly referred to as bone conduction devices, convert a received sound into vibrations. The vibrations are transferred through the skull to the cochlea causing generation of nerve impulses, which result in the perception of the received sound. Bone conduction devices are suitable to treat a variety of types of hearing loss and may be suitable for individuals who cannot derive sufficient benefit from acoustic hearing aids, cochlear implants, etc, or for individuals who suffer from stuttering problems.
US 2013/0195304 A1 relates to a transcutaneous bone conduction device vibrator having a movable magnetic mass and discloses all of the features in the preamble of claim 1.
EP2 720 480 A2 refers to magnetic spacer systems and devices for an adaption of the magnetic flux for bone conduction hearing aids.

### SUMMARY

The invention is set out in the appended claims.

### BRIEF DESCRIPTION OF THE DRAWINGS

Some embodiments are described below with reference to the attached drawings, in which:
FIG. 1 is a perspective view of an exemplary bone conduction device in which at least some embodiments can be implemented;
FIG. 2 is a schematic diagram conceptually illustrating a passive transcutaneous bone conduction device in accordance with at least some exemplary embodiments;
FIG. 3 is a schematic diagram illustrating additional details of the embodiment of FIG. 2;
FIGs. 4A-4C are schematic diagrams illustrating adjustment of a component of the embodiment of FIG. 3;
FIGs. 5A-5B are schematic diagrams illustrating exemplary magnetic flux paths of the embodiment of FIG. 3;
FIG. 6 is an exemplary chart presenting a graph representing how attraction force between the external component and the implantable component changes with relative angle change of magnets of the external component;
FIGs. 7A and 7B present an exemplary embodiment depicting how magnet orientation can be locked and limited to certain orientations;
FIG. 8 depicts another exemplary embodiment;
FIGs. 9A-10C depict other exemplary magnet configurations of the exemplary embodiment of FIG. 8; and
FIGs. 11-14 depict exemplary rotational locking concepts according to some exemplary embodiments.

### DETAILED DESCRIPTION

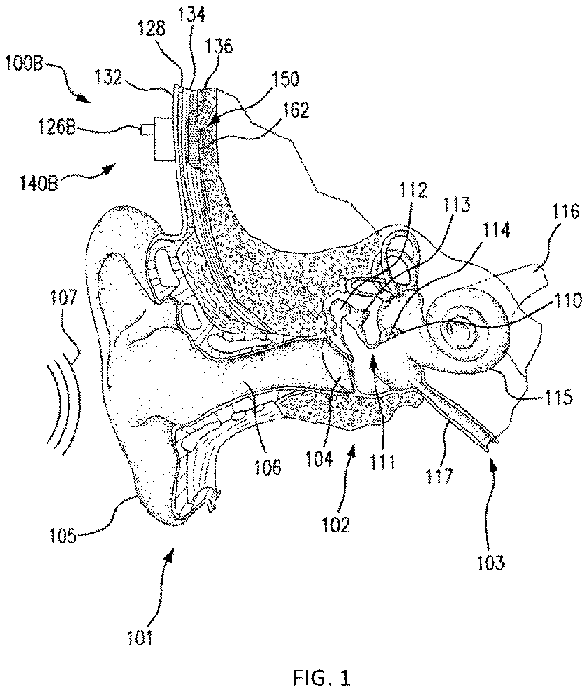

FIG. 1 is a perspective view of a bone conduction device 100 in which embodiments may be implemented. As shown, the recipient has an outer ear 101, a middle ear 102 and an inner ear 103. Elements of outer ear 101, middle ear 102 and inner ear 103 are described below, followed by a description of bone conduction device 100.

In a fully functional human hearing anatomy, outer ear 101 comprises an auricle 105 and an ear canal 106. A sound wave or acoustic pressure 107 is collected by auricle 105 and channeled into and through ear canal 106. Disposed across the distal end of ear canal 106 is a tympanic membrane 104 which vibrates in response to acoustic wave 107. This vibration is coupled to oval window or fenestra ovalis 210 through three bones of middle ear 102, collectively referred to as the ossicles 111 and comprising the malleus 112, the incus 113 and the stapes 114. The ossicles 111 of middle ear 102 serve to filter and amplify acoustic wave 107, causing oval window to vibrate. Such vibration sets up waves of fluid motion within cochlea 139. Such fluid motion, in turn, activates hair cells (not shown) that line the inside of cochlea 139. Activation of the hair cells causes appropriate nerve impulses to be transferred through the spiral ganglion cells and auditory nerve 116 to the brain (not shown), where they are perceived as sound.

FIG. 1 also illustrates the positioning of bone conduction device 100 relative to outer ear 101, middle ear 102 and inner ear 103 of a recipient of device 100. As shown, bone conduction device 100 is positioned behind outer ear 101 of the recipient and comprises a sound input element 126 to receive sound signals. Sound input element may comprise, for example, a microphone, telecoil, etc. In an exemplary embodiment, sound input element 126 may be located, for example, on or in bone conduction device 100, or on a cable extending from bone conduction device 100.

The bone conduction device 100 of FIG. 1 is a passive transcutaneous bone conduction device utilizing the electromagnetic actuators disclosed herein and variations thereof where no active component (e.g., the electromagnetic actuator) is implanted beneath the skin (it is instead located in an external device), and the implantable part is, for instance a magnetic pressure plate (a permanent magnet, ferromagnetic material, etc.). Some embodiments of the passive transcutaneous bone conduction systems are configured for use where the vibrator (located in an external device) containing the electromagnetic actuator is held in place by pressing the vibrator against the skin of the recipient. In an exemplary embodiment, the vibrator is held against the skin via a magnetic coupling (magnetic material and/or magnets being implanted in the recipient and the vibrator having a magnet and/or magnetic material that is used to complete the magnetic circuit, thereby coupling the vibrator to the recipient).

More specifically, FIG. 1 is a perspective view of a passive transcutaneous bone conduction device 100 in which embodiments can be implemented.

Bone conduction device 100 comprises an external component 140 and an implantable component 150. Bone conduction device 100 comprises a sound processor (not shown), an actuator (also not shown) and/or various other operational components. In operation, sound input device 126 converts received sounds into electrical signals. These electrical signals are utilized by the sound processor to generate control signals that cause the actuator to vibrate. In other words, the actuator converts the electrical signals into mechanical vibrations for delivery to the recipient's skull.

In accordance with some embodiments, a fixation system 162 may be used to secure implantable component 150 to skull 136. As described below, fixation system 162 may be a bone screw fixed to skull 136, and also attached to implantable component 150.

In one arrangement of FIG. 1, bone conduction device 100 is a passive transcutaneous bone conduction device. In such an arrangement, the actuator is located in external component 140, and implantable component 150 includes a plate, as will be discussed in greater detail below. The plate of the implantable component 150 vibrates in response to vibrations transmitted through the skin, mechanically and/or via a magnetic field, that are generated by an external magnetic plate.

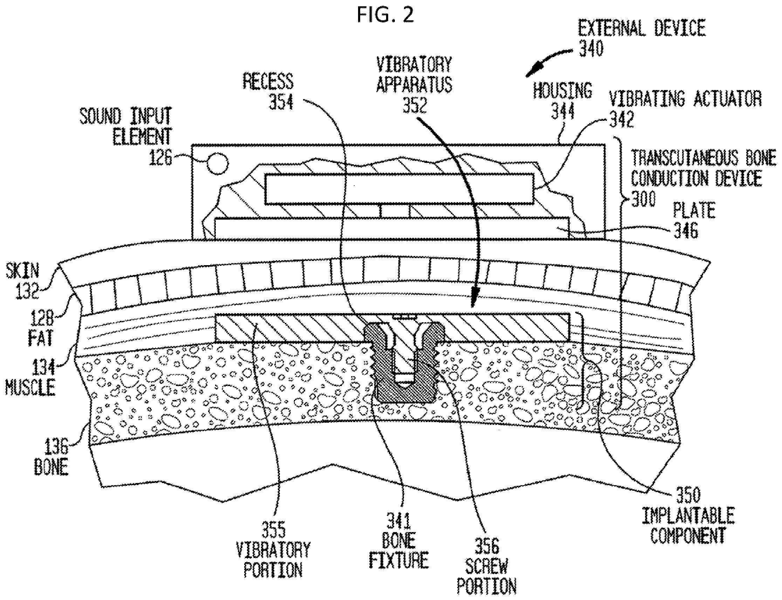

FIG. 2 depicts a functional schematic of an exemplary embodiment of a transcutaneous bone conduction device 300 according to an embodiment that includes an external device 340 (corresponding to, for example, element 140 of FIG. 1) and an implantable component 350 (corresponding to, for example, element 150 of FIG. 1). The transcutaneous bone conduction device 300 of FIG. 2 is a passive transcutaneous bone conduction device in that a vibrating electromagnetic actuator 342 is located in the external device 340. Vibrating electromagnetic actuator 342 is located in housing 344 of the external component, and is coupled to plate 346. In an exemplary embodiment, the vibrating electromagnetic actuator 342 is a device that converts electrical signals into vibration. In operation, sound input element 126 converts sound into electrical signals. Specifically, the transcutaneous bone conduction device 300 provides these electrical signals to vibrating actuator 342, or to a sound processor (not shown) that processes the electrical signals, and then provides those processed signals to vibrating electromagnetic actuator 342. The vibrating electromagnetic actuator 342 converts the electrical signals (processed or unprocessed) into vibrations. Because vibrating electromagnetic actuator 342 is mechanically coupled to plate 346, the vibrations are transferred from the vibrating actuator 342 to plate 346. Implanted plate assembly 352 is part of the implantable component 350, and is made of a ferromagnetic material that may be in the form of a permanent magnet, that generates and/or is reactive to a magnetic field, or otherwise permits the establishment of a magnetic attraction between the external device 340 and the implantable component 350 sufficient to hold the external device 340 against the skin of the recipient, as will be detailed further below. Accordingly, vibrations produced by the vibrating electromagnetic actuator 342 of the external device 340 are transferred from plate 346 across the skin to plate 355 of implanted plate assembly 352. This can be accomplished as a result of mechanical conduction of the vibrations through the skin, resulting from the external device 340 being in direct contact with the skin and/or from the magnetic field between the two plates. These vibrations are transferred without penetrating the skin with a solid object such as an abutment as detailed herein with respect to a percutaneous bone conduction device.

As may be seen, the implanted plate assembly 352 is substantially rigidly attached to a bone fixture 341 in this embodiment. Plate screw 356 is used to secure plate assembly 352 to bone fixture 341. The portions of plate screw 356 that interface with the bone fixture 341 substantially correspond to an abutment screw discussed in some additional detail below, thus permitting plate screw 356 to readily fit into an existing bone fixture used in a percutaneous bone conduction device. In an exemplary embodiment, plate screw 356 is configured so that the same tools and procedures that are used to install and/or remove an abutment screw (described below) from bone fixture 341 can be used to install and/or remove plate screw 356 from the bone fixture 341 (and thus the plate assembly 352).

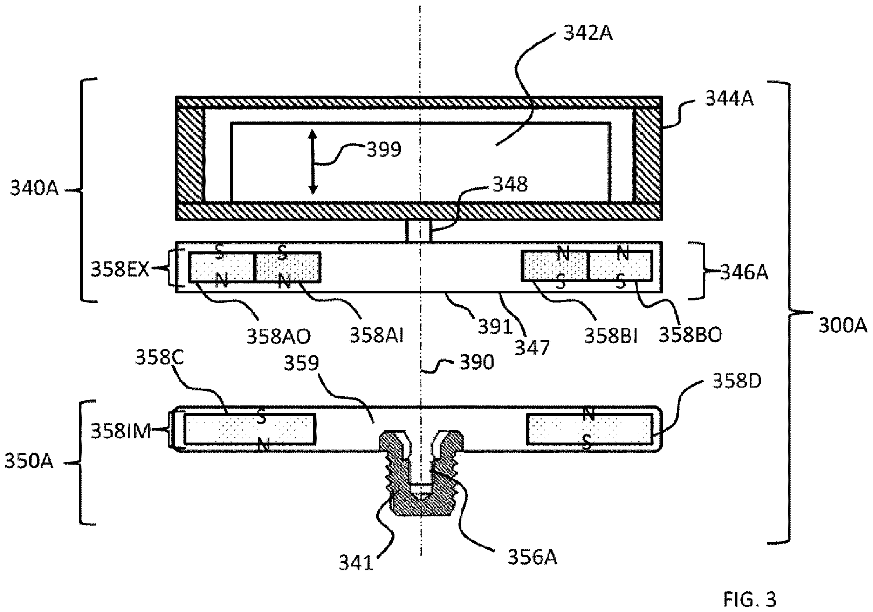

Referring now to FIG. 3, there is depicted a schematic of an exemplary bone conduction device 300A corresponding to bone conduction device 300 of FIG. 2. The exemplary bone conduction device 300A of FIG. 3 includes an external component 340A corresponding to external component 340 of FIG. 2, and an implantable component 350A corresponding to implantable component 350 of FIG. 2.

In an exemplary embodiment, external component 340A has the functionality of a transducer / actuator, irrespective of whether it is used with implantable component 350A. That is, in some exemplary embodiments, external component 340A will vibrate whether or not the implantable component 350A is present (e.g., whether or not the static magnetic field extends to the implantable component 35 OA, as will be detailed below).

The external component 340A includes a vibrating actuator represented in black-box format by reference numeral 342A. In an exemplary embodiment, the vibrating actuator can be an electromagnetic actuator. Alternatively, in some alternate embodiments, the vibrating actuator 342A can be a piezoelectric actuator. Any type of an actuator that can enable the teachings detailed herein and/or variations thereof to be practiced can be utilized in at least some exemplary embodiments. That said, embodiments detailed herein will be described, by way of example only and not by way of limitation, in terms of a vibrating electromagnetic actuator that utilizes a yoke about which is wound a coil that is energized and deenergized in an alternating manner so as to produce an electromagnetic field that interacts with permanent magnets that moves a seismic mass in a reciprocating vibratory matter in a direction of arrow 399.

Still with reference to FIG. 3, the vibrating electromagnetic actuator 342A is enclosed in a housing 344A, as can be seen. In some embodiments, the housing 344A is a hermetically sealed housing, while in other embodiments, it is not hermetically sealed. In at least some exemplary embodiments, the housing 344A is configured to provide the actuator 342A protection from shock and environmental conditions, etc. Any housing that can enable the teachings detailed herein and/or variations thereof can be utilized in at least some embodiments. In this regard, as can be seen, the housing 344A is rigidly attached to skin interface portion 346A, which functionally corresponds to plate 346 of FIG. 2 detailed above, by structural component 348. In this exemplary embodiment, the structural component 348 provides a vibrational conduction path such that vibrations generated by actuator 342 A are transferred from the housing to the skin interface component 346A such that those vibrations can then be transferred into the skin of the recipient to ultimately evoke a hearing percept according to the teachings detailed herein and/or variations thereof.

In at least some embodiments, skin interface portion 346A serves a dual role in that it both transfers vibrations from the external component 340A to the skin and also magnetically couples the external component 340A to the recipient. In this regard, as can be seen, skin interface portion 346A includes a housing 347 that includes an external magnet assembly 358EX. External magnetic assembly 358EX includes permanent magnets having a North-South alignment. These magnets are locationally adjustable relative to one another, as will be detailed below. However, in the configuration depicted in FIG. 3 (without adjustment, as will be detailed below), the magnets on one side of the magnetic assembly 358EX, relative to the longitudinal axis 390 of the bone conduction device 300A, all have North poles facing towards the actuator 342A (i.e., away from the skin of the recipient), and the magnets on the other side of the magnetic assembly 358EX, relative to longitudinal axis 390 of the bone conduction device, all have North poles facing away from the actuator 342A (i.e., towards the skin of the recipient). That is, the North-South alignment of one side of the external magnet assembly 358EX is opposite that of the other side of the assembly. However, exemplary embodiments of the external component 340A are configured such that the individual magnets can be moved so that the poles are different than that depicted in FIG. 3.

It is noted that the word "adjustable" as used herein excludes replacement of one magnet with another magnet, that being a reconfiguration or a modification to the device.

Additional details of external magnet assembly 358EX are presented below.

Skin interface portion 346A includes a bottom surface 391 (relative to the frame of reference of FIG. 3) that is configured to interface with the exterior skin of the recipient. In this regard, skin interface portion 346A corresponds to plate 346 of FIG. 2 as described above. It is through skin interface portion 346A that vibrations generated by the electromagnetic actuator of the external component 340A are transferred from the external component 340A to the skin of the recipient to evoke a hearing percept. In an exemplary embodiment, the housing 347 of the skin interface portion 346A is made of a non-ferromagnetic material that is compatible with skin of the recipient (or at least is coated with a material that is compatible with skin of the recipient). In this regard, in at least some exemplary embodiments, the housing 347 is configured to substantially avoid influencing the magnetic flux generated by the permanent magnets of the external magnet assembly 358EX.

FIG. 3 also depicts an implantable component 350A corresponding to implantable component 350 of FIG. 2. In some embodiments, implantable component 350 includes an implantable magnet assembly 358IM that includes at least two permanent magnets 358C and 358D. Permanent magnet 358C has a North-South alignment in a first direction relative to a longitudinal axis of the electromagnetic actuator (the vertical direction of FIG. 3). Permanent magnet 358D has a North-South alignment in a second direction relative to a longitudinal axis of the electromagnetic actuator, the second direction being opposite the first direction. In an exemplary embodiment, the permanent magnets are bar magnets (having a longitudinal direction extending normal to the plane of FIG. 3). In at least some exemplary embodiments, permanent magnets 358C and 358D are bar magnets connected to one another via the chassis 359 of the implantable component 350A. In an exemplary embodiment, the chassis 359 is a nonmagnetic material (e.g., titanium). It is noted that in alternative embodiments, other configurations of magnets can be utilized. Any configuration permanent magnet that can enable the teachings detailed herein and/or variations thereof to be practiced can be utilized in at least some embodiments.

That said, it is noted that the implantable component 350A does not include permanent magnets. In at least some embodiments, elements 358C and 358D are replaced with other types of ferromagnetic material (e.g., soft iron (albeit encapsulated in titanium, etc.)). Also, elements 358C and 358D can be replaced with a single, monolithic component. Any configuration of ferromagnetic material of the implantable component 35 OA that will enable the permanent magnets of the external component 340A to establish a magnetic coupling with the implantable component 350A that will enable the external component 340A to be adhered to the surface of the skin, as detailed herein, can be utilized in at least some embodiments.

As can be seen, implantable component 35 OA includes screw component 356A configured to screw into bone fixture 341 and thus secure the chassis 359 to the bone fixture 341, and thus to the recipient.

Referring back to the external component 340A, and, more particularly, to the external magnetic assembly 358EX of the skin interface portion 346A, it can be seen that the external magnetic assembly 358EX comprises four (4) different magnets arrayed about the longitudinal axis 390 in two sets. The first set includes outer permanent magnet 358AO and inner permanent magnet 358AI. The second set includes outer permanent magnet 358BO and inner permanent magnet 358BI. As will be detailed more thoroughly below, the inner permanent magnets of these sets are configured to be moved relative to the outer permanent magnets of the sets, and/or visa-versa, so as to vary the magnetic flux generated by the external magnetic assembly 358EX as a result of magnetic flux addition and cancellation. In this regard, in at least some exemplary embodiments, during operational use of the bone conduction device 300A, the magnets of the external magnet assembly 358EX are aligned with the magnets of the implantable magnet assembly 358IM such that the poles of the permanent magnets 358AO, 358AI and 358C have a North-South alignment in the same direction and the poles of the permanent magnets 358BO, 359BI and 358D have a North-South alignment in the same direction (but opposite of that of magnets 358AO, 358AI and 358C), in a scenario where maximum attractive force between the external component 340A and the implantable component 350A is desired. Conversely, in at least some exemplary embodiments, during operational use of the bone conduction device 300A, the magnets of the external magnet assembly 358EX are aligned with the magnets of the implantable magnet assembly 358IM such that the poles of the permanent magnets 358AO and/or 358AI are aligned in a different direction than that of magnet 358C, not because the external component 340A has been rotated relative to the implantable component 350A (or, alternatively, not entirely because the external component 340A has been rotated relative to the implantable component 35 OA), but because of the adjustability of the relative position of the magnets 358AO and/or 358AI. Furthermore, in this exemplary embodiment, during operational use of the bone conduction device 300A, the magnets of the external magnet assembly 358EX are aligned with the magnets of the implantable magnet assembly358IM such that the poles of the permanent magnets 358BO and/or 358BI are aligned in a different direction than that of magnet 358D, again not because the external component 340A has been rotated relative to the implantable component 35 OA (or, alternatively, not entirely because the external component 340A has been rotated relative to the implantable component 350A), but because of the adjustability of the relative position of the magnets 358BO and/or 358BI.

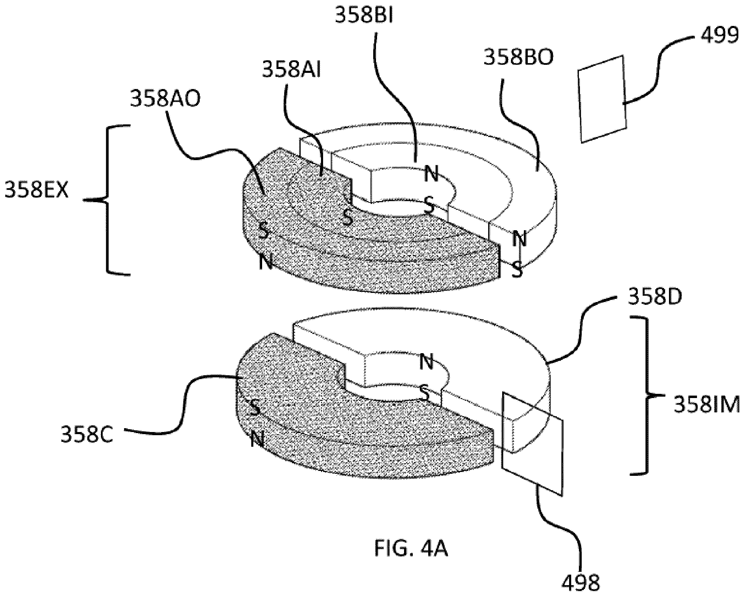

The above adjustability can be conceptually seen in FIGs. 4A-C, which conceptually depict respective isometric views of the external magnet assembly and the internal magnet assembly without any of the components of the bone conduction device 300A. More specifically, FIG. 4A depicts a configuration of the external magnet assembly 358EX such that the maximum attraction force between the external component 340A and 350A is achieved. Briefly, with respect to the frame of reference of FIGs. 4A-4C, the plane 499 corresponds to the plane of FIG. 3, wherein the plane 499 lies on the longitudinal axis 390 of the bone conduction device 300A. Plane 498 is perpendicular to plain 499, and also lies on the longitudinal axis 390 of the bone conduction device 300A. Plane 499 is in the middle of magnets 358C and 358D and in the middle of magnets 358AO and 358BO, and in the middle of magnets 358AI and 359BI, at least when all of those magnets are perfectly aligned axially and radially with respect to one another, and thus, along with longitudinal axis 390, defines the orientation of plane 499 relative to the bone conduction device 300A.

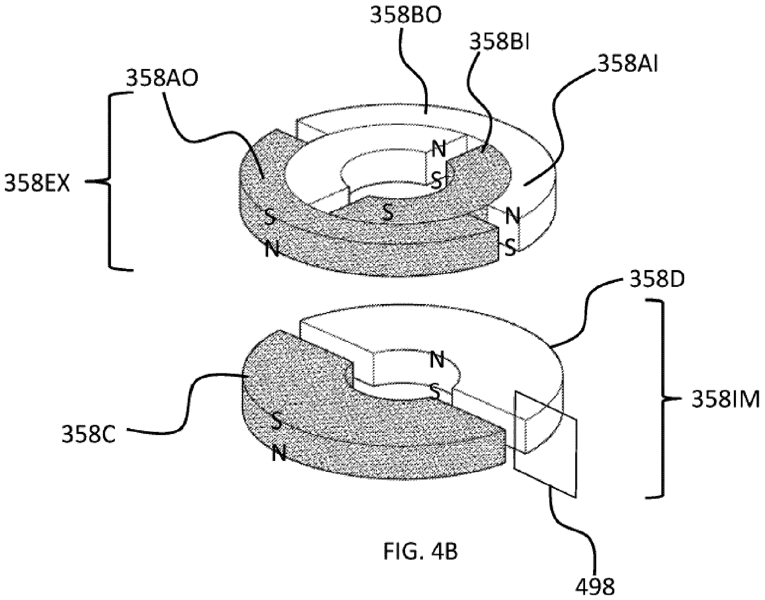

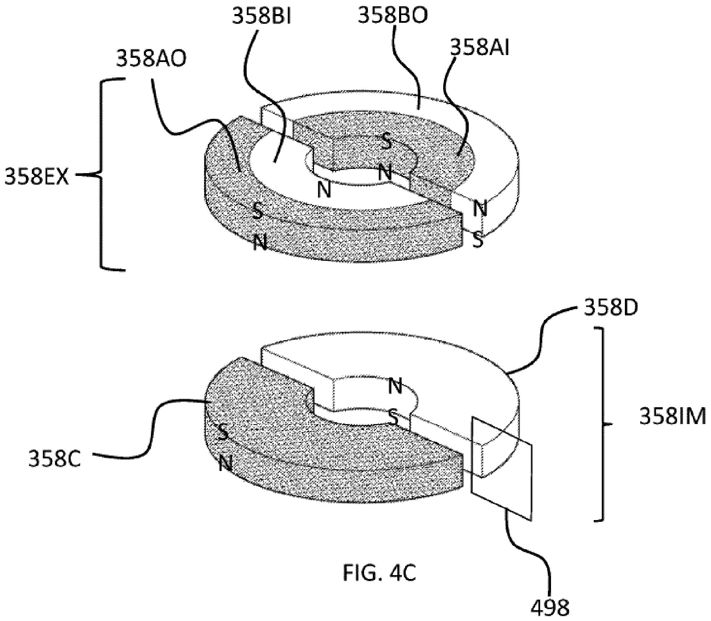

As can be seen in FIG. 4A, the magnets of the external component are segmented into 2 half-washer shaped magnets of approximately equal area. In embodiments corresponding to FIGs. 4A-4C, the external component 340A is configured such that the inner magnets 358AI and 358BI can be moved to have a different angular configuration relative to the outer magnets 358AO and 358BO. Accordingly, FIG. 4B depicts the inner magnets 358AI and 358BI shifted by an angle 90 degrees relative to the location of those magnets depicted in FIG. 4A (and thus having an angular offset of 90 degrees relative to the outer magnets 358AO and 358BO). Here, and in FIG. 4C, the locations of outer magnets 3 58AO and 358BO are the same as that of FIG. 4A. Also, the locations of outer magnets 358AO and 358BO, relative to the magnets of the implantable magnet assembly 358IM, are the same in all of FIGs. 4A-4C. It is noted that the arrangement of FIG. 4A depicts four (4) magnets in the external component (aside from any magnets that may be present in, for example, the transducer). That is, even though the poles of two of the four magnets are aligned with one another and those two magnets are contacting one another, that "set" of magnets still represents two magnets. That is, a given magnet is a discrete magnet, and while a plurality of magnets aligned with one another function as a single magnet, there are still a plurality of magnets present.

FIG. 4C depicts the inner magnets 358AI and 358BI shifted by an angle 180 degrees relative to the location of those magnets depicted in FIG. 4A (and thus having an angular offset of 180 degrees relative to the outer magnets 358AO and 358BO). In these embodiments, whereas the configuration of FIG. 4A results in the strongest attraction force (for a given air gap between the external magnet assembly 358EX and the implantable magnet assembly 358IM - more on this below) between the external component 340A and the implantable component 350A, the configuration of FIG. 4C results in the weakest attraction force (again for the given air gap) between the external component 340A and the implantable component 350, with the configuration of FIG. 4B resulting in an attraction force between that resulting from FIG. 4A and FIG. 4C.

The physical phenomenon that results in the differences between the attraction force of the different configurations will now be described, followed by some exemplary embodiments of the structure of the bone conduction device implementing some such embodiments.

Briefly, a general concept of an exemplary principle of operation here is that a net attractive force between the external component and the implanted component is needed to maintain the external component against the skin of the recipient. An attractive force of zero would result in the external component not being retained to the recipient, at least via magnetic attraction, and a negative attractive force would repel the external component from the implantable component. However, the net attractive force can be varied within a range, providing that a net attractive force still remains, and the embodiments detailed herein can enable that variation. That is, in at least some exemplary embodiments the magnets are adjusted to vary the net attractive force.

In this regard, as will be discussed in greater detail below, it is noted that even with the magnets located as positioned in FIG. 4C, there is still some net attractive force remaining between the external component and the implantable component, even though the magnets are locally oppositely arranged with respect to their poles. This is because, in this exemplary embodiment, the outer magnets dominate the inner magnets with respect to the net magnetic attraction. That is, the outer magnets have a greater effect on the magnetic attraction than that of the inner magnets. That said, in at least some embodiments, if the inner magnets had a more dominant effect on the overall net magnetic attraction, the movements of the magnets could possibly result in a net repulsive force (or if the outer magnets were the magnets that were adjusted). In at least most embodiments, there is utilitarian value with respect to maintaining an overall net magnetic attraction, at least in scenarios where a zeroed out net magnetic attraction and/or a net repulsive attraction would not result in the external component being maintained in position against the skin of the recipient.

Accordingly, there can be utilitarian value with respect to the teachings detailed herein and variations thereof in also taking into account the local effect of a given magnet (i.e., the effect of a magnet on the overall system). In this regard, some magnets can generate a magnetic field that is stronger than other magnets, and also the positioning of magnets (including the distance of magnets of the external component to the implantable component) can influence the overall effect of the magnets with respect to the net attractive force between the external component and the implantable component.

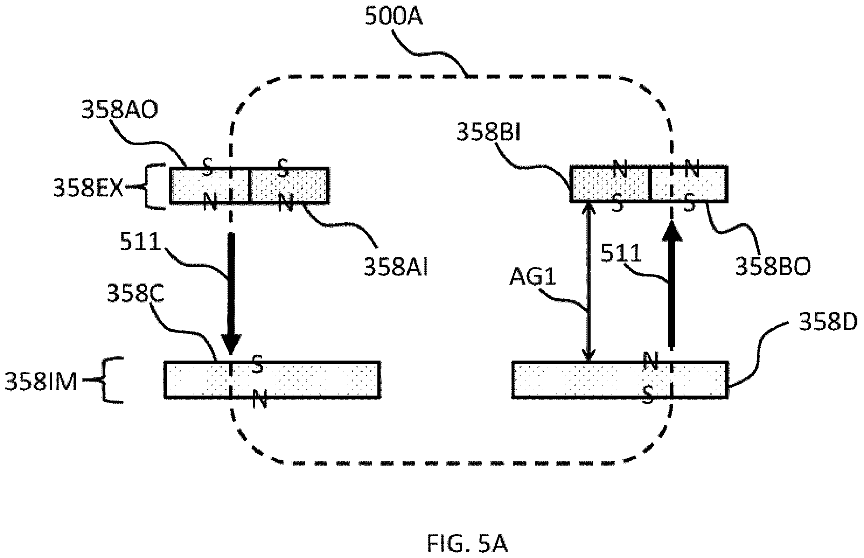

FIG. 5A depicts a quasi-functional diagram of a cross-section of the external and implantable magnet assemblies taken through plane 499 (the plane of FIG. 3) with the magnets in the arrangement as presented in FIG. 4A, with the magnetic flux following a magnetic flux path 500A. FIG. 5A depicts an air gap AG1, representing the space between the external magnet assembly 358EX and the implantable magnet assembly 358IM. It is noted that the phrase "air gap" refers to locations along the magnetic flux path in which little to no material having substantial magnetic aspects is located but the magnetic flux still flows through the gap. The air gap closes the magnetic field. Accordingly, an air gap is not limited to a gap that is filled by air. Indeed, in at least some embodiments, there is always some form of solid and/or liquid matter located between the opposing faces of the external and internal magnet assemblies (skin, fat, body fluids, the material of the chassis 359, material of the housing 347, etc.).

As can be seen, in an exemplary embodiment, the magnetic flux path 500A travels in a circuit through all of the magnets of the external magnet assembly and the implantable magnet assembly. Arrows 511 depict the relative localized strength of the magnetic flux and the direction thereof in between the magnets of the external magnet assembly 358EX and the implantable magnet assembly 358IM (the strength and direction of the magnetic flux at those local locations within the air gap AG1). With respect to the cross-sectional view of FIG. 5A, the magnetic flux travels in a counterclockwise direction, as is represented by arrows 511, consistent with the fact that the poles of all the magnets are aligned. That said, if the view of FIG. 5 A was presented from the opposite side of the longitudinal axis 390, the direction of the magnetic flux would be clockwise. Alternatively, if all of the magnetic poles were reversed from that seen in the figures, the magnetic flux would be in the opposite direction.

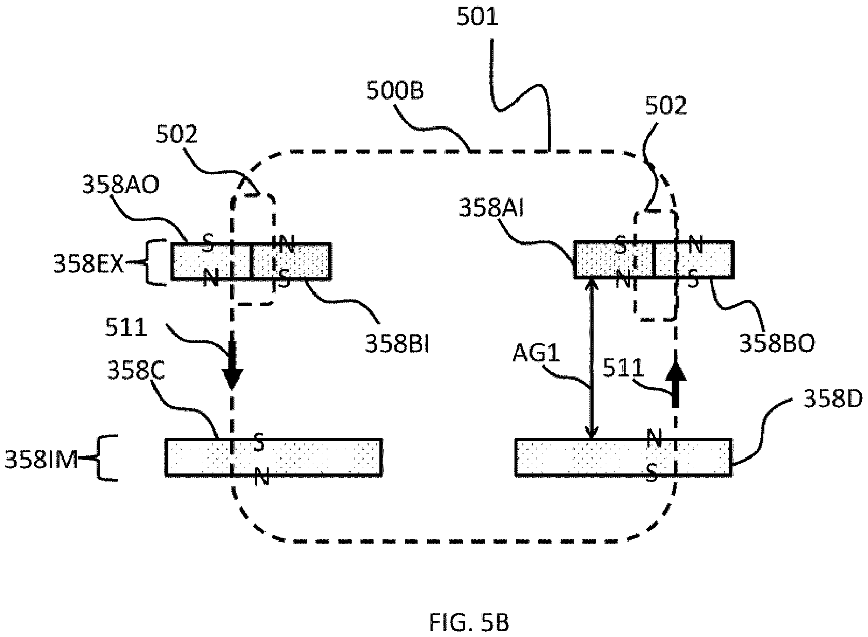

Conversely, FIG. 5B depicts a quasi-functional diagram of a cross-section of the external and implantable magnet assemblies also taken through plane 499 (the plane of FIG. 3) with the magnets in the arrangement as presented in FIG. 4C, with the magnetic flux path 500B superimposed thereon. FIG. 5B depicts an air gap AG1, representing the space between the external magnet assembly 358EX and the implantable magnet assembly 358IM. The air gap AG1 is the same distance as that of FIG. 5 A in this exemplary embodiment.

As can be seen, in an exemplary embodiment the magnetic flux has a magnetic flux path 500B that includes multiple components. First, a flux path 501 that travels in a circuit through all of the magnets of the external magnet assembly and the implantable magnet assembly, at least generally concomitant with the flux path 500A of FIG. 5A vis-a-vis directionality. However, as can be seen, the magnetic flux path 500B also includes flux paths 502 that travel in a circuit only through the local magnets on either side of the longitudinal axis 390 of the bone conduction device 300A. That is, as can be seen, there is a magnetic flux path 502 that travels in a localized circuit between magnets 358AO and 358BI, owing to the fact that the directionality of the poles of these magnets are opposite relative to the longitudinal axis 390. The same is the case with respect to magnets 358BO and 358AI. Generally speaking, the magnetic flux paths 502 represent a short circuit in the magnetic flux path 500A of FIG. 5 A that limits that magnetic interaction between the magnets of the external magnet assembly 358EX and the magnets of the implantable magnet assembly 358IM relative to that which would be the case in the absence of the short-circuiting. In at least some embodiments, because of these localized magnetic flux paths 502, the strength of the magnetic flux between the magnets of the external magnet assembly 358EX and the implantable magnet assembly 358IM (the strength within the air gap AG1) is lower relative to that of FIG. 5A. This is functionally represented by arrows 51 1 in FIG. 5B, which are smaller than those of FIG. 5 A. Accordingly, the resulting retention force holding the external component 350A against the skin of the recipient is lower than the resulting retention force of the magnetic flux of FIG. 5A.

Accordingly, in at least some exemplary embodiments, the bone conduction device 300A is configured such that the strength of the magnetic field generated (at least in part) by the external component can be varied for a given air gap AG1 and a given orientation of the external component 340A relative to the implantable component 350A, by establishing a short-circuit of the magnetic flux and controlling the magnitude of that short-circuit. That is, by way of example only and not by way of limitation, holding all other variables constant, the magnetic flux that retains the external component 340A to the implantable component 350A can be varied such that the resulting retention force that holds the external component 340A to the skin of the recipient is also varied by adjusting the orientation of at least one permanent magnet of the external component 340A relative to another permanent magnet of the external component 340A, and thereby creating and/or adjusting the short-circuit in the magnetic flux.

Thus, in view of the above, in an exemplary embodiment, there is an apparatus, comprising an external component of a medical device, such as, by way of example only and not by way of limitation, external component 340 of FIG. 3, configured to generate a magnetic flux (e.g., via permanent magnets, which generation is thus passive) that removably retains, via a resulting magnetic retention force, the external component (e.g., 340A) to the recipient thereof. In this exemplary embodiment, the external component is configured to enable the adjustment of the generated magnetic flux so as to vary the resulting magnetic retention force. In this regard, as seen from the above, at least some exemplary embodiments accomplish this by moving the permanent magnets relative to one another. In this regard, in at least some exemplary embodiments, the external component is configured to enable the adjustment of the generated magnetic flux without varying a total magnetic density of permanent magnets of the external component generating the magnetic flux. That said, in at least some alternate embodiments, as will be detailed below, permanent magnets can be added and/or removed from the external component of the bone conduction device so as to vary the generated magnetic flux, and thus vary the force retaining the external component to the recipient.

Still further, as will be understood from the embodiment of FIG. 3, in at least some exemplary embodiments, the external component is configured to enable the adjustment of the generated magnetic flux entirely due to the generation of passive magnetic flux (e.g., the magnetic flux generated by permanent magnets, as contrasted to that generated by the application of electric current to a coil, etc.). As just-detailed, this can be accomplished without varying a total magnetic density of permanent magnets of the external component generating the magnetic flux.

Still further, as will be understood from the above, in an exemplary embodiment of this exemplary embodiment, the external component is configured to enable the adjustment of the generated magnetic flux via at least one of additive or subtractive interaction of local magnetic flux (e.g., by creating and/or varying the short-circuit in the magnetic flux, by, for example, altering / adjusting the relative locations of one or more of the permanent magnets that generate the magnetic field).

In at least some specific exemplary embodiments, the external component 340A includes at least a first permanent magnet and a second permanent magnet (e.g., 358BI and 358BO, respectively), and the external component is configured to enable the adjustment of the generated magnetic flux via movement of the first permanent magnet relative to the second permanent magnet (or visa-versa, or by movement of both the first permanent magnet and the second permanent magnet). As seen above with respect to FIGs. 4A-4C, the movement of the first permanent magnet relative to the second permanent magnet is movement within a single plane (e.g., the plane normal to the longitudinal axis 390 of the bone conduction device 300A). Still further, as seen above with respect to FIGs. 4A-4C, a first permanent magnet relative to the second permanent magnet is a rotational movement within that plane. That said, in alternative embodiments, as will be detailed below, the movement need not necessarily be rotational.

Before proceeding further to some of the performance features of at least some exemplary embodiments, it is briefly noted that in at least some exemplary embodiments, the external component 340A includes a sound processor. In at least some embodiments, at least one of the magnets (e.g., 358AI and/or 358BI) of the external component 340A is movable relative to the sound processor.

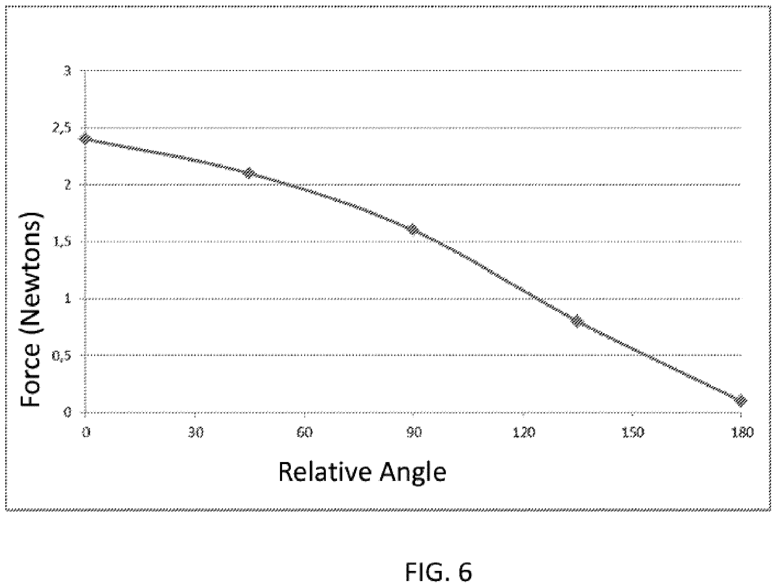

FIG. 6 presents a chart that depicts an exemplary graph of attraction force in Newtons between the external components 340A and the implantable component 350A for relative angle adjustment between the magnets of the external magnet assembly 358EX, where zero degrees corresponds to the orientation of the magnets seen in FIG. 4A, 90 degrees corresponds to the orientation of the magnets seen in FIG. 4B, and 180 degrees corresponds to the orientation of the magnets seen in FIG. 4C, where all variables other than angular orientation of the permanent magnets of the external component 340A relative to one another are held constant. Further, it is noted that the exemplary forces depicted in FIG. 6 are for a given magnet configuration. Stronger magnets and/or larger magnets, etc., would result in different force values for the relative angles. Accordingly, the data depicted in FIG. 6 is exemplary to illustrate a general concept for some embodiments. That said, the data is accurate for other embodiments.

As can be seen from the graph of FIG. 6, in at least some embodiments, the external magnet assembly 358EX is configured such that the attraction force between the external component 340A and the implantable component 350A can be varied such that the attraction force can be reduced to approximately 10% of the maximum attraction force (i. e., the force at the zero angle of alignment). It is noted that in at least some embodiments, the external magnet assembly 358EX is configured such that the attraction force between the external component 340A and the implantable component 350A can be varied such that the attraction force can be reduced to approximately 75%, 70%, 65%, 60%, 55%, 50%, 45%, 40%, 35%, 30%, 25%, 20%, 15%, 10% or less of the maximum attraction force or about any value there between in about 1% increments (e.g., about 64%, about 17%, etc.).

Thus, in view of the above, in an exemplary embodiment, the external component 340A (or any other external component detailed herein and/or variations thereof or others based thereon, that can enable the teachings detailed herein and/or variations thereof) is configured to enable the adjustment of a generated magnetic flux generated at least in part by the external component, so as to vary the resulting magnetic retention force between the external component and the implantable component, solely due to the adjustment of the generated magnetic flux, from a maximum retention force (all other variables held constant) to a retention force that is less than any of about 75%, 70%, 65%, 60%, 55%, 50%, 45%, 40%, 35%, 30%, 25%, 20%, 15% or about 10% of the maximum force, or any value there between as detailed above.

Any force and any relative angle and any relationship there between that can enable the teachings detailed herein to be practiced (e.g., retaining an external component of a bone conduction device to a recipient to evoke a hearing percept) can be utilized in at least some embodiments.

As noted above, the inner magnets of the external magnet assembly 358EX (magnets 358AI and 358BI) are moved relative to the outer magnets and to the other components of the external component 340A of the bone conduction device 300A. Conversely, in alternative embodiments, it is the outer magnets of the external magnet assembly 358EX (magnets 358AO and 358BO) that are moved relative to the inner magnets and to the other components of the external component 340A of the bone conduction device 300A. Still further, in some other alternate embodiments, both the outer and inner magnets are moved relative to the other components of the external component 340A of the bone conduction device 300A.

In at least one exemplary embodiment, a mechanical arrangement is utilized to move the magnets relative to one another. In an exemplary embodiment, a worm gear is utilized to rotate the inner magnets relative to the outer magnets and/or vice-versa. To this end, in an exemplary embodiment, the inner magnets of the external magnet assembly 358EX are arrayed in/on a structure such that the inner magnets are connected to one another (the magnets can be connected to a circular plate of non-magnetic material, the magnets can be embedded in a casting of nonmagnetic material in the form of a ring, etc.). The structure can include or otherwise be connected to a rotary gear that interfaces with a worm gear. The external component 340A is configured such that a torque can be applied to the worm gear such that the torque turns the worm gear, which in turn turns the rotary gear included/connected to the structure in which/on which the inner magnets are arrayed, thereby changing the angular relationship between the inner magnets and the outer magnets. In an alternative embodiment, the outer magnets can be located in/on the structure to which the rotary gear is connected, and thus torque applied to the worm gear results in the rotation of the outer magnets, thereby changing the angular relationship between the outer magnets and the inner magnets. In still other alternate embodiments, the external component 340A of the bone conduction device 300A is configured such that a single worm gear rotates both the outer magnets and the inner magnets to change the angular orientation of the respective magnets.

In at least some exemplary embodiments, the external component 340A of the bone conduction device can be configured to permit a compact power tool to be connected to the external component such that the worm gear (or any other gearing system that is utilized) can be turned at a higher speed than that which can be achieved by turning the worm gear by hand. In this regard, the gearing system of at least some exemplary embodiments can be configured such that the gearing system converts a high-speed input to a low speed and high torque output.

In at least some embodiments, precise angular positioning of the outer magnets relative to the inner magnets and/or vice versa can have utilitarian value. In at least some embodiments, the external component 340A of the bone conduction device is configured such that the angular orientation of the magnets relative to one another can be changed in increments of 0.5°, 1°, 2°, 3°, 4°, 5°, 6°, 7°, 8°, 9°, 10°, 15°, 20°, 30°, 45° or more or any value or range of values there between in 0.1° increments (e.g, 0.7° increments, 1.5° increments, etc.). Accordingly, in an exemplary embodiment, a high degree of precision with respect to force adjustment can be achieved, because the angular relationship between the magnets can be adjusted to such a fine and precise degree that very fine and precise changes in the attraction force can be obtained.

Other mechanical configurations can be utilized to change the angular orientations of the magnets. In an exemplary embodiment, the outer magnets and/or the inner magnets are connected to one another by structure such that a torque applied to the structure will change the angular orientation of the magnets relative to one another. In an exemplary embodiment, the outer magnets and/or the inner magnets can be located on a flat plate and/or be embedded in a structure, analogous to the structures detailed above with respect to the gearing system, and a torque application point can be provided on that structure. In an exemplary embodiment, the torque application point can be a hex head receptacle that will interface with an Allen wrench or the like. The external component 340A of the bone conduction device is configured such that application of a torque to the torque point utilizing the Allen wrench can change the angular orientation of the magnets.

In an alternative embodiment, there is a method of changing the angular orientation of the magnets relative to one another by utilizing an external fixture that generates an external magnetic field that has sufficient strength such that it repositions the magnets relative to one another. In an exemplary embodiment, this can be utilized in embodiments where the magnets of the external magnet assembly 358EX are completely enclosed within the housing 347 (e.g., hermetically sealed therein) and there is no adjustment mechanism built into the external component 340A. By way of example only and not by way of limitation, the outer magnets 358AO and 358BO can be fixed to the housing 347, and the inner magnets 358AI and 358BI can be arranged such that the magnets can move when the magnetic field is applied thereto. Alternatively, the inner magnets can be fixed to the housing 347, and the outer magnets can be configured to move when the magnetic field is applied thereto.

Of course, in at least some embodiments, the relative angle of the magnets can be adjusted by hand. By way of example only and not by way of limitation, the outer magnets and/or the inner magnets can be located in or on any of the structure(s) detailed above, and those structure(s) can be connected to a component that is accessible from the outside of the housing 347. In an exemplary embodiment, this component can be a ring that has a knurled surface that extends about the outer circumference of the housing 347 and is movable relative thereto. In an exemplary embodiment, a recipient or a healthcare professional or technician, or anyone suitable to practice at least some of the teachings detailed herein, rotates the knurled ring relative to the housing 347. Because the ring is connected to the structure supporting the movable magnets, the pertinent magnets are moved with the ring.

Still further, in at least some embodiments, the external component 340A can simply be taken apart to gain access to some or all of the magnets of the external magnet assembly 358EX, and the pertinent magnets can be adjusted by hand to the desired angular orientation.

Any device, system, and/or method that will enable the angular orientation of one or more of the permanent magnets of the external magnet assembly 358EX to be adjusted that will enable the teachings detailed herein and or variations thereof to be practiced can be utilized in at least some embodiments.

At least some embodiments also include a structural arrangement that will enable the magnets to be secured in place after the desired adjustment is achieved. Any configuration that will enable the magnets to be retained at a desired angular orientation / "locked in place" after movement can be utilized in at least some embodiments. By way of example only and not by way of limitation, a lock screw can be utilized to prevent the magnets from rotating relative to one another after angular adjustment. In an exemplary embodiment, the outer magnets 358AO and 358BO of the external magnet assembly 358EX and/or the inner magnets 358AI and 358BI of the external magnet assembly 358EX can be supported on/in any of the structures detailed above. For example, the magnets can be located within rings of nonmagnetic material, such that an outer ring containing the outer magnets is arrayed about an inner ring (or disk) containing the inner magnets. A lock screw can extend through the outer ring to the inner ring. Friction force between the tip of the lock screw and the outer surface of the inner ring can be used to hold the inner ring in place such that its angular orientation relative to the outer ring will not change. Alternatively and/or in addition to this, the lock screw can extend in a direction parallel to the longitudinal axis 390, thus bypassing the outer ring.

While friction force between the tip of the lock screw and a given ring is utilized to hold the magnets in position in some embodiments, dimpled sections can be utilized to receive a portion of the tip of the lock screw in alternative embodiments, the dimpled portions can be arrayed about the outer circumference of the outer ring in discrete intervals such that discrete angular orientations of the inner magnets relative to the outer magnets can be maintained.

It is noted that while the above embodiments focus on rotating the inner magnets relative to the outer magnets, in an alternate embodiment, it is the outer magnets that are rotated. Accordingly, in an exemplary embodiment, the lock screw need not extend to the inner magnets / the ring supporting the inner magnets. Instead, the lock screw would, in at least some embodiments, extend only to the outer magnets / the ring supporting the outer magnets.

That said, in at least some embodiments, friction forces are utilized to hold the magnets in place, if not lock the magnets in place. In an exemplary embodiment, the magnets will not move unless a sufficient torque or force is applied thereto to overcome the friction. Alternatively and/or in addition to this, the external component may be configured such that the magnets are always free to move relative to one another, but a significant amount of input must be provided to move the magnets relative to one another. By way of example only and not by way limitation, in an embodiment that utilizes gearing, the external component can be configured such that many, many turns must be applied to the input to rotate the magnet just one degree. Thus, even if a limited number of turns are ultimately applied to the input, the magnet will not move a significant amount.

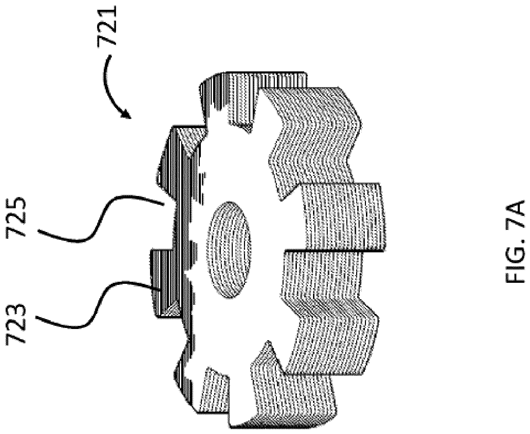

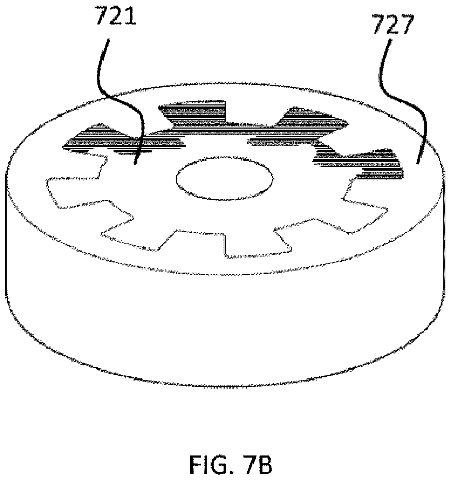

An exemplary embodiment can utilize a tongue and recess system to maintain a desired angular orientation between the magnets. Referring now to FIG. 7A, there is an exemplary casting 721 of a plastic material containing the inner magnets (not shown). As can be seen, the casting 721 includes eight (8) tongues 723 and eight (8) recesses 725. The casting 721 is sized and dimensioned to fit into a casting 727 having the opposite configuration, as can be seen in FIG. 7B. In an exemplary embodiment, this casting 727 includes the outer magnets (not shown). As can be seen, there are eight (8) combinations of angular orientations of the casting 721 relative to casting 727, and thus the inner magnets relative to the outer magnets. That said, in at least some embodiments, there are only three different resulting force profiles in at least some of these embodiments, because the resulting force values of orientations of the magnets after the 180° relative orientations can be duplicative of those before the 180° orientation. Accordingly, in an exemplary embodiment, the embodiment of FIGs. 7A and 7B enables the relative orientation of the outer magnets to the inner magnets to be changed in 45° increments (i.e., the angular orientation of the magnets can be set to 0°, 45° degrees, 90° degrees, 135° degrees, 180°, and so on until the 0° orientation is again obtained). In at least some embodiments, the embodiments of FIGs. 7A and 7B can have utilitarian value in that a very precise angular orientation of the magnets can be maintained with very high reliability (e.g., in at least some embodiments, the only way for an orientation to change after adjustment of the magnets would be if the housing 347 of the external component 340A was purposely taken apart and/or the housing 347 was damaged).

It is noted that while the embodiments described above utilize rotation of magnets relative to one another to alter the strength of the magnetic field between the magnets of the external magnet assembly and the implantable magnet assembly, other modes of moving the magnets relative to one another to vary the strength of the magnetic field can be utilized. By way of example only and not by way of limitation, the sliding movement/translational movement of the magnets relative to one another can be utilized, providing that such will result in the varying of the strength of the magnetic field (e.g. by creating and or varying the magnetic flux short circuit, etc.). That said, there are other embodiments that can utilize rotation and/or other movement of magnets relative to one another.

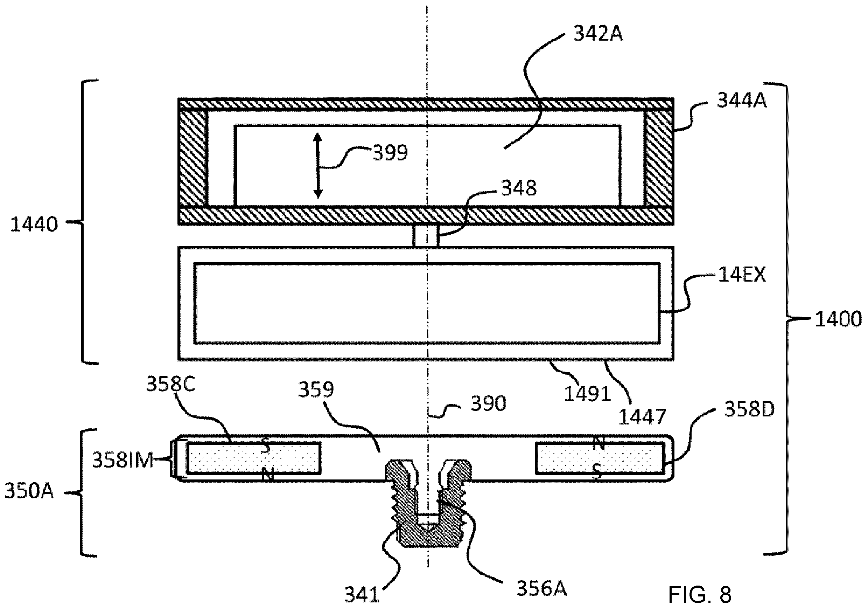

FIG. 8 depicts a schematic of another exemplary bone conduction device 1400 corresponding to bone conduction device 300 of FIG. 2. In this exemplary embodiment, the implantable component 350A of bone conduction device 1400 is the same as that of the bone conduction device 300A. Conversely, the external component 1440 has a different configuration than that of external component 340A. Briefly, the permanent magnets that generate, at least in part, the magnetic flux that is utilized to retain the external component 1400 to the recipient are located within black box 14EX in a stacked manner (relative to the longitudinal axis 390). Additional details of this configuration will now be provided.

External component 1440 has the functionality of a transducer / actuator, irrespective of whether it is used with implantable component 350A. The external component 1440 includes a vibrating actuator represented in black-box format by reference numeral 342A.

External component 1440 includes an external magnet assembly that includes permanent magnets having a North-South alignment. These magnets are locationally adjustable relative to one another, as will be detailed below.

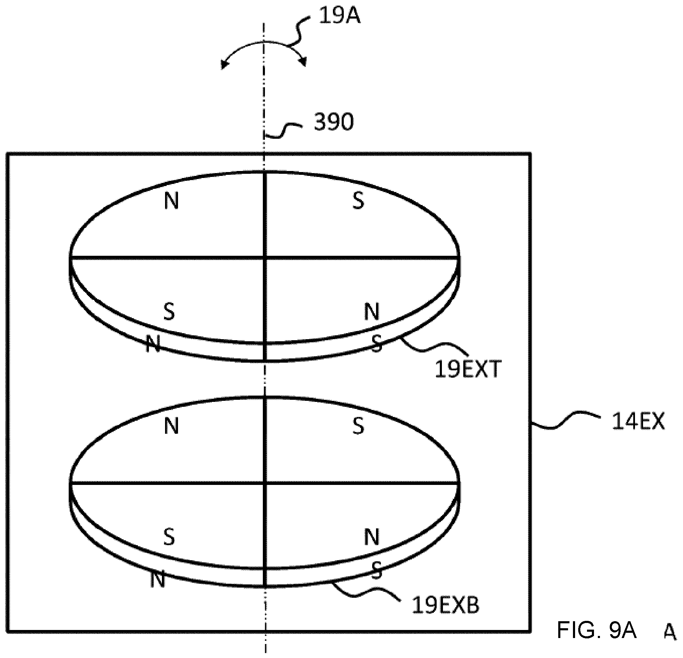

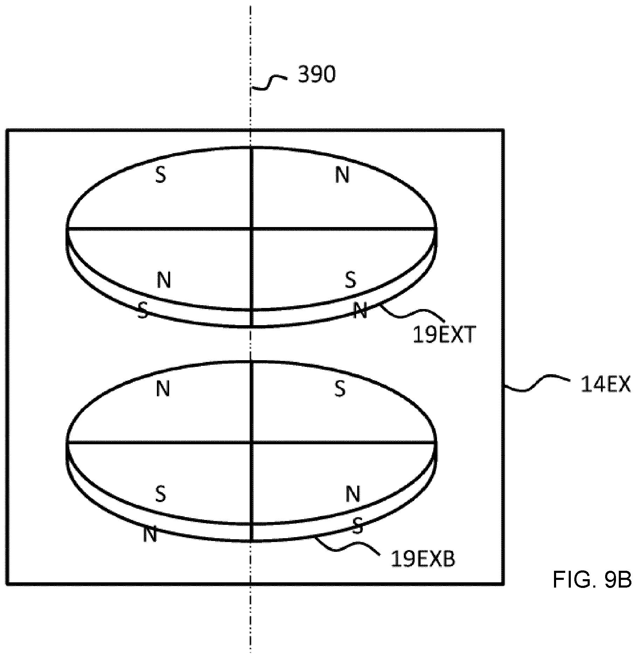

In some alternate embodiments, there are two or more segmented magnets within 14EX. For example, FIGs. 9A and 9B depict such a configuration, including top segmented magnet 19EXT and bottom segmented magnet 19EXB. In the embodiment of FIG. 9A, one or both of the magnets are rotatable relative to one another so as to vary the resulting magnetic field, and thus vary the strength of the resulting attraction force between the external component and the implantable component.

In view of the above, in an exemplary embodiment, there is a bone conduction device, including a first permanent magnet, a second permanent magnet, a third permanent magnet and a fourth permanent magnet, wherein the first permanent magnet (any of the segments of magnet 19EXT) is movable relative to the second permanent magnet and the fourth permanent magnet (any of the segments of magnet 19EXB) so as to adjust a strength of a magnetic field resulting from the first and second permanent magnets, and the third permanent magnet (any of the segments of magnet 19EXT) is movable relative to the fourth permanent magnet and the second permanent magnet so as to adjust the strength of the magnetic field.

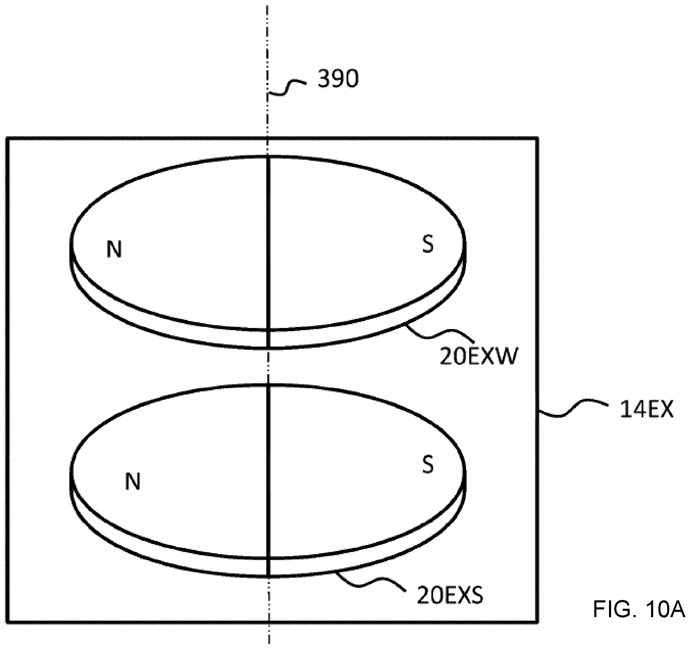

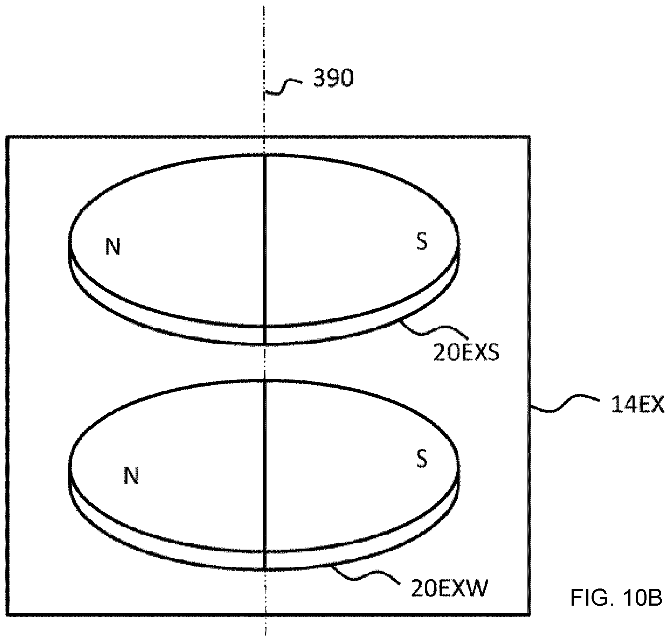

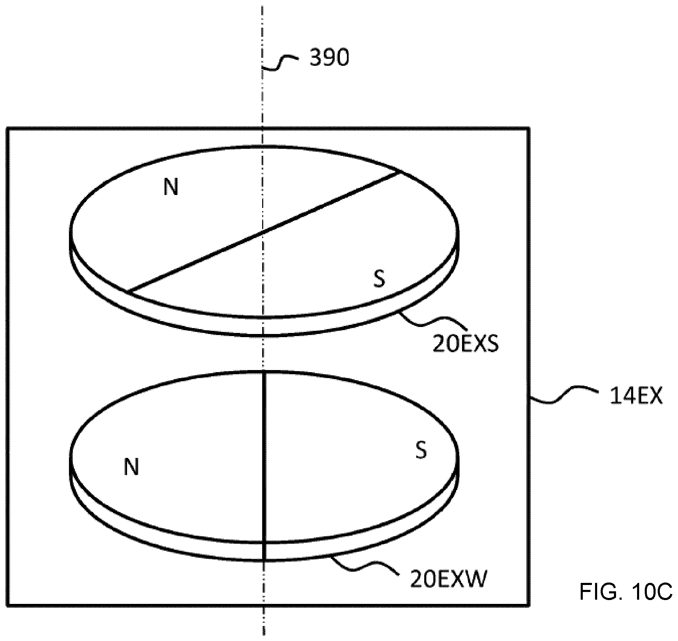

As detailed above, some embodiments utilize magnets that generate stronger magnetic fields relative to other magnets utilized in the external component of the bone conduction device. FIG. 10 depicts an exemplary embodiment that utilizes magnets of the same size that generate magnetic fields of different strengths. This can be achieved through use of different magnetic materials, degree of magnetization etc. In this regard, 14EX includes a top magnet 20EXW and a bottom magnet 20EXS, both of which are segmented into half-moon segments having opposite polarities on the top and bottom surfaces. (It is noted that the concepts of this embodiment can be implemented utilizing at least some of the other magnet configurations detailed herein (e.g., a magnet segmented into 4 sections, such as those above). The top magnet 20EXW is a weak magnet, and the bottom magnet 12EXS is a strong magnet, relative to one another. In an exemplary embodiment, bone conduction device is configured such that the order of the magnets with respect to the stack direction can be varied, as can be seen by comparing FIG. 10A to FIG. 10B. By way of example only and not by way of limitation, by moving the strong magnet 20EXS further away from the implantable component and moving the weak magnet 20EXW closer to the implantable component, the retention force between the external component and the implantable component is reduced relative to that which was the case with the magnets in the reversed order.

It is also noted that the embodiments of FIGs. 10A and 10B are such that the magnets can be replaced with different magnets having different strengths, and such that the magnets can be rotated relative to one another. With regard to this latter embodiment, FIG. 10C depicts the order in which the magnets in 14EX are stacked are both reversed relative to that which is the case in FIG. 10A and with one of the magnets, the strong magnet 20EXS, rotated about the longitudinal axis 390 relative to its orientation in the configuration of FIG. 10B. Alternatively and/or in addition to this, the weak magnet 20EXW can be rotated relative to its orientation in the configuration of FIG. 10B.

In view of the above, it can be seen that in an exemplary embodiment, permanent magnets can be utilized to allow for a range of adjustment of the retention force between the external component and the implantable component with little to no increase in the size of the external component for a given magnet configuration relative to that which would be the case if the magnets were not adjustable. This is as compared to an external component where open/unused space must be made available for a permanent magnet to be moved therein to vary the resulting retention force, wherein open space must be provided for the magnet to enter.

It is noted that while the embodiments detailed herein are directed towards a passive transcutaneous bone conduction device in general, and providing a magnetic coupling for an external component of a passive transcutaneous bone conduction device in particular, other embodiments can utilize other types of prostheses, such as cochlear implants, active transcutaneous bone conduction devices and middle ear / DACI devices, at least with respect to the transcutaneous communication components thereof. By way of example only and not by way of limitation, the teachings detailed herein can be applicable to a so-called "button sound processor." That is an exemplary embodiment includes an external component that includes microphone(s), a sound processor, and potentially other functional components, and that is held to a recipient via magnetic attraction with an implantable component according to the teachings detailed herein and/or variations thereof, where the button sound processor provides a signal to an implanted (implantable) component (e.g., a component including, for example, an implanted transducer (e.g., electromagnetic actuator), implanted cochlear stimulator, implanted middle-ear actuator, etc.), such signal being, for example, an electromagnetic signal (e.g., a signal provided by an inductance link) transmitted from the button sound processor to the implanted (implantable) component.

In view of the above, it can be seen that the teachings detailed herein and or variations thereof, in at least some embodiments, can be utilized to customize the retention force for a given recipient. This can be done with respect to the long-term (e.g., simply developing a retention force that is comfortable for the recipient for future use, where a "one size fits all" approach is achieved (or "two sizes fit all" or "one size fits many" approach is achieved)) and with respect to the short-term (e.g. permitting the retention force to be adjusted depending on a given circumstances, such as for example increasing the retention force when the recipient is jogging or otherwise engaging in an activity resulting in higher G forces than normal use, etc.). As can be seen, in at least some embodiments, this can enable the adjustment, in at least some embodiments, without the need for other extra components / extra parts to be placed into or added to the external component. That is, the retention force can be adjusted utilizing only the components that are provided with a given external component (albeit tools may be utilized to adjust the force - it is just that there are no extra and/or alternate parts of the hearing prosthesis that are needed to accomplish the adjustment).

In at least some embodiments, this can have utilitarian value with respect to avoiding necrosis and/or reducing the likelihood of necrosis. That said, it can be seen that in at least some embodiments, the range of adjustments of the resulting force can be over a range such that the high-end is about an order of magnitude stronger than the low-end. In this regard, in at least some embodiments, the adjustment mechanism could permit too strong of a force to be applied, if only by accident (this is as compared to a device where only one setting exists). Accordingly, in at least some embodiments, there can be utilitarian value in limiting the range of adjustments of the magnets, so as to limit the range of retention force that can result in the adjustment of the magnets. Such exemplary embodiments will be described in terms of the rotational magnet arrangement. That said, it is noted that the following concepts can be applied to other embodiments detailed herein and/or variations thereof.

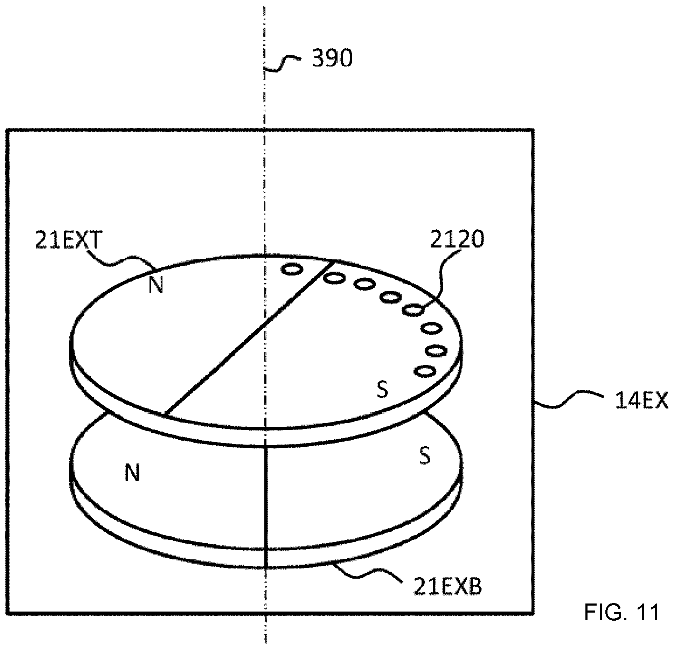

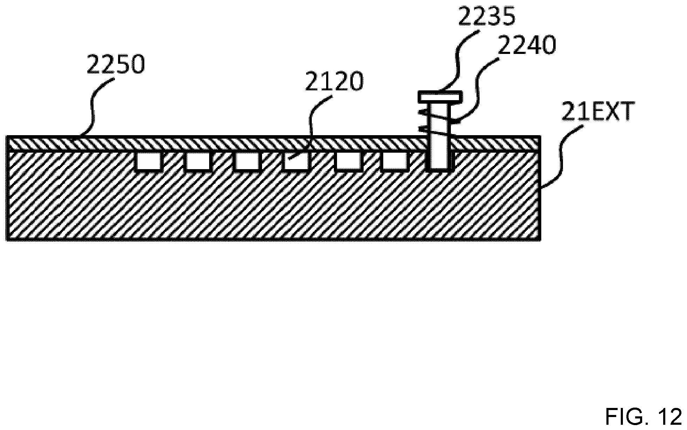

Referring now to FIG. 11, an arrangement can be seen where 14EX includes a top magnet 21EXT and a bottom magnet 21EXB, or at least the top magnet 21EXT is rotatable relative to the bottom magnet 21EXB. As can be seen, there is an array of holes 2120 located in the top surface of the top magnet 21 EXT. In an exemplary embodiment of the external component 1440, a spring-loaded lock-pin 2235 interfaces with the holes 2120 on a selective basis so as to lock the location of the top magnet relative to the bottom magnet. This can be seen in FIG. 12, which is an extrapolated cross-sectional view through 14EX taken in a direction parallel to the longitudinal axis 390. As can be seen, a spring 2240 is connected to the lock-pin 2235. In an exemplary embodiment, the lock-pin 2235 is spring-loaded to be forced downward into a hole 2120 though the housing wall 2250. During use, a user pulls on the head of the pin 2235 to pull the pin out of the hole 2120 so that the magnet 20EXT can be rotated.

Because the holes are arrayed only in a limited pattern, the top magnet 21EXT can only be locked at certain angular locations relative to the bottom magnet 21EXB. In the locations where the holes are not present, the magnet 21EXT cannot be locked. Thus, this prevents the magnet from being locked at a location where the resulting retention force is too strong (or, alternatively, too weak, in some other embodiments). That is, while the 20EXT can be rotated to a location where the resulting attraction force may be too strong, it cannot be locked in place at that location. That said, in some alternative embodiments, the external component 1440 can be configured such that the external component cannot be used unless lock-pin 2235 is engaged in one of the holes 2120.

It is noted that this embodiment is applicable to any embodiment where it is desired to lock the movable magnet in place after the adjustment and not just those where the range of adjustment is desired to be limited.

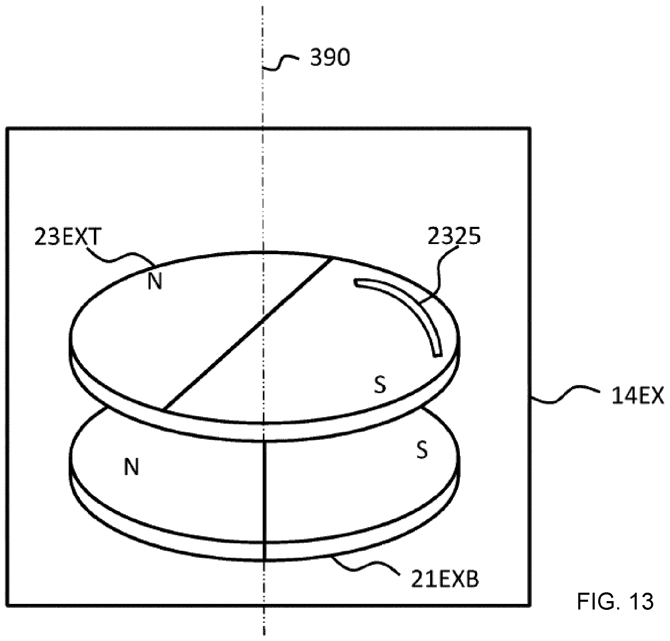

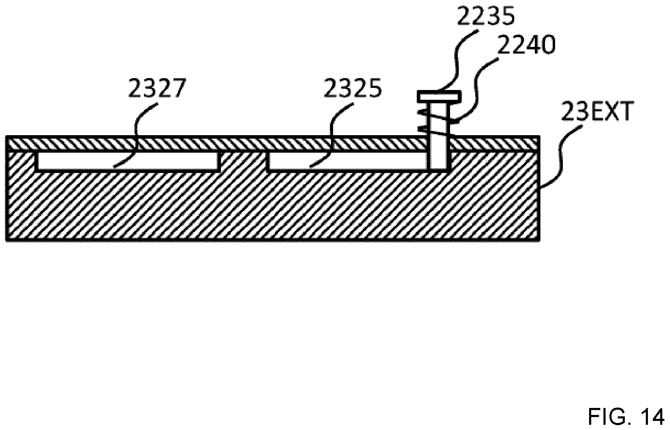

Figure 13 provides an alternate embodiment of the concept of FIG. 11. Here, instead of separate holes, a slot 2325 is located in the top surface of the top magnet 23EXT. FIG. 14 provides a cross-sectional view of this concept, being taken in a plane that is parallel to the longitudinal axis 390. Here, two slots can be seen: slots 2325, and 2327. In an exemplary embodiment, the top magnet 23EXT can be rotated only over a range between the end walls of the slot 2325 (if the pin 2235 is located therein) or the slit 2327 (if the pin 2235 is located therein). This is because the end walls of the slots will hit pin 2235 unless the pin is withdrawn from the slot. This can enable the forced to be adjusted over the range of movements within a given slot, while requiring the pin to be affirmatively removed from a slot to adjust the magnet 23 EXT outside the range of a given slot.

As noted above, some and/or all of the teachings detailed herein can be used with a passive transcutaneous bone conduction device. Thus, in an exemplary embodiment, there is a passive transcutaneous bone conduction device including one or more or all of the teachings detailed herein that is configured to effectively evoke hearing percept. By "effectively evoke a hearing percept," it is meant that the vibrations are such that a typical human between 18 years old and 40 years old having a fully functioning cochlea receiving such vibrations, where the vibrations communicate speech, would be able to understand the speech communicated by those vibrations in a manner sufficient to carry on a conversation provided that those adult humans are fluent in the language forming the basis of the speech. In an exemplary embodiment, the vibrational communication effectively evokes a hearing percept, if not a functionally utilitarian hearing percept.

With regard to methods, an exemplary method entails obtaining an external component of a medical device configured to be magnetically retained against outer skin of a recipient via a magnetic coupling between the external component and an implanted component in the recipient (e.g., the embodiment of FIGs 3 etc.) and adjusting an orientation of one or more magnets of the external component relative to at least one other magnet of the external component (e.g., moving the magnet as detailed with respect to the embodiment of FIG. 3), such that the resulting retention force of the magnetic retention for the recipient is varied from that which was the case prior to the adjustment. Still further, in some embodiments, this method is executed such that the retention force is varied such that at least one of a 25% reduction in the force occurs or a 25% increase in the force occurs, for the recipient, and/or or that the action of adjusting a location of one or more magnets of the external component at least one of creates a short-circuit or varies an existing short-circuit of the magnetic flux between the external component and the implantable component, thereby varying the resulting retention force.

Still further, in at least some embodiments, the action of adjusting the orientation of one or more magnets of the external component such that the resulting retention force is varied is executed without changing a total magnetic density of permanent magnets of the external component and/or the action of adjusting a location of one or more magnets of the external component such that the resulting retention force is varied is executed without changing a total magnetic density of permanent magnets of the external component and/or the action of adjusting the location of one or more magnets of the external component such that the resulting retention force is varied is executed without changing a global position of a magnet of the external component. In at least some embodiments, the action of adjusting the orientation of one or more magnets of the external component such that the resulting retention force is varied is executed without removing or adding any magnets to the external component and/or the action of adjusting the orientation of one or more magnets of the external component is executed without directly accessing the one or more magnets from outside the external component.

In at least some embodiments, any one or more or all of the teachings detailed herein are applicable to the implantable component(s) detailed herein. It is further noted that in some embodiments, both the implantable component and the external component can utilize the adjustable features detailed herein.

## Claims

1. An apparatus, comprising:
an external component of a medical device configured to generate a magnetic flux so that the external component can be removably retained to a recipient thereof, via a resulting magnetic retention force between the external component and an implanted magnet of the recipient, wherein the external component is configured to enable the adjustment of the generated magnetic flux so as to vary the resulting magnetic retention force,
wherein the external component includes at least a first permanent magnet and a second permanent magnet, and wherein the external component is configured to enable the adjustment of the generated magnetic flux, wherein the external component is configured for repositioning the first permanent magnet relative to the second permanent magnet via a movement of the first permanent magnet relative to the second permanent magnet,
**characterized in that** the external component is configured to allow movement of the first permanent
magnet relative to the second permanent magnet in a single plane normal to a longitudinal axis (390),
wherein the external component comprises a skin interface portion (346A) including a bottom surface (391) that is configured to interface with the exterior skin of the recipient so that the longitudinal axis (390) is perpendicular to the bottom surface (391).

2. The apparatus of claim 1, wherein:
the external component is configured to enable the adjustment of the generated magnetic flux via at least one of additive or subtractive interaction of local magnetic flux.

3. The apparatus of claim 1, wherein:
the external component is configured such that the repositioning of the first permanent magnet relative to the second permanent magnet occurs entirely within the confines of the external component.

4. The apparatus of claim 1, wherein:
the repositioning of the first permanent magnet relative to the second permanent magnet is a rotational movement.

5. The apparatus of claim 1, wherein:
the external component is configured to enable the adjustment of the generated magnetic flux so as to vary the resulting magnetic retention force, solely due to the adjustment of the generated magnetic flux, from a maximum retention force to a retention force that is less than 30% of the maximum force.

6. The apparatus of claim 1, wherein:
the external component includes a worm- gear mechanism configured to reposition the first permanent magnet relative to the second permanent magnet.

7. The apparatus of claim 1, wherein:
the external component includes a wrench interface configured to reposition the first permanent magnet relative to the second permanent magnet upon the application of a torque thereto.

8. The apparatus of claim 1, wherein:
the apparatus is a bone conduction device, wherein
the first permanent magnet is movable relative to the second permanent magnet so as to adjust a strength of a magnetic field resulting from the first and second permanent magnets.

9. The apparatus of claim 8, wherein:
the first permanent magnet is rotatable relative to the second permanent magnet so as to adjust the strength of the magnetic field.

10. The apparatus of claim 8, further comprising:
a third permanent magnet and a fourth permanent magnet, wherein
the magnetic field also results from the third and fourth permanent magnets, the third permanent magnet is movable relative to the fourth permanent magnet and the second permanent magnet so as to adjust the strength of the magnetic field, and
the first permanent magnet is movable relative to the fourth permanent magnet so as to adjust the strength of the magnetic field resulting from the first, second third and fourth permanent magnets.

11. The apparatus of claim 10, wherein:
the first and third permanent magnets are segments of a segmented top magnet (19EXT),
the second and fourth permanent magnets are segments of a segmented bottom magnet (19EXB).

12. The apparatus of claim 10, wherein:
the first and third permanent magnets are arrayed in a first circular path;
the second and fourth permanent magnets are arrayed in a second circular path encompassing or encompassed by the first circular path, and
the first and third permanent magnets move along the first circular path and thus relative to the second and fourth permanent magnets so as to adjust the strength of the magnetic field resulting from the first, second third and fourth permanent magnets,
wherein:
the first and second circular paths are arrayed about an axis of rotation,
relative to a plane normal to the axis of rotation, the alignment of the poles of the first and second permanent magnets are the same and the alignment of the poles of the third and fourth permanent magnets are also the same and opposite of that of the first and second permanent magnets.

13. The apparatus of claim 8, wherein:
the first permanent magnet is movable relative to the second permanent magnet such that the alignment of poles of the first permanent magnet relative to those of the second permanent magnet are reversed so as to decrease the strength of the magnetic field resulting from the first and second permanent magnets.

14. The apparatus of claim 8, wherein: the bone conduction device includes a sound processor; and
the bone conduction device is configured such that the first magnet is movable relative to the sound processor and the second magnet is locational fixed relative to the sound processor.

## Patentansprüche

1. Vorrichtung, die Folgendes umfasst:
eine externe Komponente einer medizinischen Vorrichtung, die so konfiguriert ist, dass sie einen magnetischen Fluss erzeugt, so dass die externe Komponente über eine resultierende magnetische Haltekraft zwischen der externen Komponente und einem implantierten Magneten des Empfängers lösbar an diesem gehalten werden kann, wobei die externe Komponente so konfiguriert ist, dass sie die Einstellung des erzeugten magnetischen Flusses ermöglicht, um die resultierende magnetische Haltekraft zu variieren,
wobei die externe Komponente mindestens einen ersten Dauermagneten und einen zweiten Dauermagneten umfasst, und wobei die externe Komponente so konfiguriert ist, dass sie die Einstellung des erzeugten Magnetflusses ermöglicht, wobei die externe Komponente so konfiguriert ist, dass sie den ersten Dauermagneten relativ zum zweiten Dauermagneten über eine Bewegung des ersten Dauermagneten relativ zum zweiten Dauermagneten neu positionieren kann,
**dadurch gekennzeichnet, dass**
die externe Komponente so konfiguriert ist, dass sie eine Bewegung des ersten Permanentmagneten relativ zu dem zweiten Permanentmagneten in einer einzigen Ebene senkrecht zu einer Längsachse (390) ermöglicht,
wobei die externe Komponente einen Hautschnittstellenabschnitt (346A) umfasst, der eine Bodenfläche (391) enthält, die so konfiguriert ist, dass sie mit der Außenhaut des Empfängers eine Schnittstelle bildet, so dass die Längsachse (390) senkrecht zu der Bodenfläche (391) verläuft.

2. Vorrichtung nach Anspruch 1, wobei:
das externe Bauteil so konfiguriert ist, dass es die Einstellung des erzeugten magnetischen Flusses durch mindestens eine additive oder subtraktive Wechselwirkung des lokalen magnetischen Flusses ermöglicht.

3. Vorrichtung nach Anspruch 1, wobei:
das externe Bauteil so konfiguriert ist, dass die Neupositionierung des ersten Permanentmagneten relativ zu dem zweiten Permanentmagneten vollständig innerhalb der Grenzen des externen Bauteils erfolgt.

4. Vorrichtung nach Anspruch 1, wobei:
die Neupositionierung des ersten Permanentmagneten relativ zum zweiten Permanentmagneten eine Rotationsbewegung ist.

5. Vorrichtung nach Anspruch 1, wobei:
die externe Komponente so konfiguriert ist, dass sie die Einstellung des erzeugten magnetischen Flusses ermöglicht, um die resultierende magnetische Haltekraft allein aufgrund der Einstellung des erzeugten magnetischen Flusses von einer maximalen Haltekraft zu einer Haltekraft zu variieren, die weniger als 30% der maximalen Kraft beträgt.

6. Vorrichtung nach Anspruch 1, wobei:
die externe Komponente einen Schneckenradmechanismus umfasst, der so konfiguriert ist, dass er den ersten Permanentmagneten relativ zum zweiten Permanentmagneten neu positioniert.

7. Vorrichtung nach Anspruch 1, wobei:
die externe Komponente eine Schnittstelle für einen Schraubenschlüssel aufweist, die so konfiguriert ist, dass sie den ersten Dauermagneten relativ zum zweiten Dauermagneten neu positioniert, wenn ein Drehmoment auf ihn aufgebracht wird.

8. Vorrichtung nach Anspruch 1, wobei:
die Vorrichtung eine Knochenleitungsvorrichtung ist, wobei
der erste Dauermagnet relativ zum zweiten Dauermagneten beweglich ist, um die Stärke eines Magnetfeldes einzustellen, das sich aus dem ersten und zweiten Dauermagneten ergibt.

9. Vorrichtung nach Anspruch 8, wobei:
der erste Dauermagnet relativ zum zweiten Dauermagneten drehbar ist, um die Stärke des Magnetfeldes einzustellen.

10. Vorrichtung nach Anspruch 8, die ferner umfasst:
einen dritten Dauermagneten und einen vierten Dauermagneten, wobei
das Magnetfeld auch aus dem dritten und vierten Permanentmagneten resultiert, der dritte Permanentmagnet relativ zu dem vierten Permanentmagneten und dem zweiten Permanentmagneten beweglich ist, um die Stärke des Magnetfeldes einzustellen, und
der erste Permanentmagnet relativ zum vierten Permanentmagneten beweglich ist, um die Stärke des Magnetfeldes einzustellen, das sich aus dem ersten, zweiten, dritten und vierten Permanentmagneten ergibt.

11. Vorrichtung nach Anspruch 10, wobei:
der erste und der dritte Dauermagnet Segmente eines segmentierten oberen Magneten (19EXT) sind,
der zweite und der vierte Dauermagnet Segmente eines segmentierten unteren Magneten (19EXB) sind.

12. Vorrichtung nach Anspruch 10, wobei:
der erste und der dritte Dauermagnet auf einer ersten Kreisbahn angeordnet sind;
der zweite und der vierte Dauermagnet in einer zweiten Kreisbahn angeordnet sind, die die erste Kreisbahn umschließt oder von ihr umschlossen wird, und
der erste und der dritte Dauermagnet sich entlang der ersten Kreisbahn und damit relativ zu dem zweiten und dem vierten Dauermagneten bewegen, um die Stärke des von dem ersten, dem zweiten, dem dritten und dem vierten Dauermagneten erzeugten Magnetfeldes einzustellen,
wobei:
die erste und die zweite Kreisbahn um eine Drehachse angeordnet sind,
die Ausrichtung der Pole des ersten und zweiten Permanentmagneten in Bezug auf eine Ebene senkrecht zur Drehachse gleich ist und die Ausrichtung der Pole des dritten und vierten Permanentmagneten ebenfalls gleich und entgegengesetzt zu der des ersten und zweiten Permanentmagneten ist.

13. Vorrichtung nach Anspruch 8, wobei:
der erste Dauermagnet relativ zum zweiten Dauermagneten beweglich ist, so dass die Ausrichtung der Pole des ersten Dauermagneten relativ zu denen des zweiten Dauermagneten umgekehrt wird, um die Stärke des aus dem ersten und zweiten Dauermagneten resultierenden Magnetfeldes zu verringern.

14. Vorrichtung nach Anspruch 8, wobei: die Knochenleitungsvorrichtung einen Soundprozessor enthält; und
die Knochenleitungsvorrichtung so konfiguriert ist, dass der erste Magnet relativ zu dem Soundprozessor beweglich ist und der zweite Magnet relativ zu dem Soundprozessor ortsfest ist.

## Revendications

1. Appareil comprenant :
un composant externe d'un dispositif médical configuré pour générer un flux magnétique afin que le composant externe puisse être retenu de manière amovible à un destinataire, grâce à une force de rétention magnétique résultante entre le composant externe et un aimant implanté du destinataire, dans lequel le composant externe est configuré pour permettre l'ajustement du flux magnétique généré de manière à faire varier la force de rétention magnétique résultante,
dans lequel le composant externe comprend au moins un premier aimant permanent et un deuxième aimant permanent, et dans lequel le composant externe est configuré pour permettre l'ajustement du flux magnétique généré, dans lequel le composant externe est configuré pour repositionner le premier aimant permanent par rapport au deuxième aimant permanent via un mouvement du premier aimant permanent par rapport au deuxième aimant permanent,
**caractérisé par le fait que**
le composant externe est configuré pour permettre le mouvement du premier aimant permanent par rapport au deuxième aimant permanent dans un seul plan normal à l'axe longitudinal (390),
dans lequel le composant externe comprend une partie d'interface avec la peau (346A) comprenant une surface inférieure (391) configurée pour être en interface avec la peau extérieure du receveur de sorte que l'axe longitudinal (390) soit perpendiculaire à la surface inférieure (391).

2. Appareil selon la revendication 1, dans lequel :
le composant externe est configuré pour permettre l'ajustement du flux magnétique généré par au moins une interaction additive ou soustractive du flux magnétique local.

3. Appareil selon la revendication 1, dans lequel :
le composant externe est configuré de telle sorte que le repositionnement du premier aimant permanent par rapport au deuxième aimant permanent se produit entièrement dans les limites du composant externe.

4. Appareil selon la revendication 1, dans lequel :
le repositionnement du premier aimant permanent par rapport au deuxième aimant permanent est un mouvement de rotation.

5. Appareil selon la revendication 1, dans lequel :
le composant externe est configuré pour permettre le réglage du flux magnétique généré de manière à faire varier la force de rétention magnétique résultante, uniquement due au réglage du flux magnétique généré, d'une force de rétention maximale à une force de rétention inférieure à 30 % de la force maximale.

6. Appareil selon la revendication 1, dans lequel :
le composant externe comprend un mécanisme à vis sans fin configuré pour repositionner le premier aimant permanent par rapport au deuxième aimant permanent.

7. Appareil selon la revendication 1, dans lequel :
le composant externe comprend une interface de clé configurée pour repositionner le premier aimant permanent par rapport au deuxième aimant permanent lors de l'application d'un couple.

8. Appareil selon la revendication 1, dans lequel :
l'appareil est un dispositif de conduction osseuse, dans lequel
le premier aimant permanent est mobile par rapport au deuxième aimant permanent de manière à ajuster l'intensité d'un champ magnétique résultant des premier et deuxième aimants permanents.

9. Appareil selon la revendication 8, dans lequel :
le premier aimant permanent peut tourner par rapport au deuxième aimant permanent afin de régler l'intensité du champ magnétique.

10. Appareil selon la revendication 8, comprenant en outre
un troisième aimant permanent et un quatrième aimant permanent, dans lequel
le champ magnétique résulte également des troisième et quatrième aimants permanents, le troisième aimant permanent est mobile par rapport au quatrième aimant permanent et au deuxième aimant permanent de manière à ajuster l'intensité du champ magnétique, et
le premier aimant permanent est mobile par rapport au quatrième aimant permanent de manière à ajuster l'intensité du champ magnétique résultant des premier, deuxième, troisième et quatrième aimants permanents.

11. Appareil selon la revendication 10, dans lequel :
les premier et troisième aimants permanents sont des segments d'un aimant supérieur segmenté (19EXT),
les deuxième et quatrième aimants permanents sont des segments d'un aimant inférieur segmenté (19EXB).

12. Appareil selon la revendication 10, dans lequel :
les premier et troisième aimants permanents sont disposés selon une première trajectoire circulaire ;
les deuxième et quatrième aimants permanents sont disposés selon une deuxième trajectoire circulaire qui englobe ou est englobée par la première trajectoire circulaire, et
les premier et troisième aimants permanents se déplacent le long de la première trajectoire circulaire et donc par rapport aux deuxième et quatrième aimants permanents de manière à ajuster l'intensité du champ magnétique résultant des premier, deuxième, troisième et quatrième aimants permanents,
dans lequel :
les première et deuxième trajectoires circulaires sont disposées autour d'un axe de rotation,
par rapport à un plan normal à l'axe de rotation, l'alignement des pôles des premier et deuxième aimants permanents est identique et l'alignement des pôles des troisième et quatrième aimants permanents est également identique et opposé à celui des premier et deuxième aimants permanents.

13. Appareil selon la revendication 8, dans lequel :
le premier aimant permanent est mobile par rapport au deuxième aimant permanent de sorte que l'alignement des pôles du premier aimant permanent par rapport à ceux du deuxième aimant permanent est inversé de manière à diminuer l'intensité du champ magnétique résultant des premier et deuxième aimants permanents.

14. Appareil selon la revendication 8, dans lequel :
le dispositif de conduction osseuse comprend un processeur de son ; et
le dispositif de conduction osseuse est configuré de manière à ce que le premier aimant soit mobile par rapport au processeur de son et que le deuxième aimant soit fixe par rapport au processeur de son.
